## Europäisches Patentamt
## European Patent Office
## Office européen des brevets

(19)

(11) Veröffentlichungsnummer : **0 039 010**
**B1**

(12) **EUROPÄISCHE PATENTSCHRIFT**

(45) Veröffentlichungstag der Patentschrift :
26.10.83

(51) Int. Cl.³ : **A 61 F 5/01**, A 61 F 13/06

(21) Anmeldenummer : **81102945.3**

(22) Anmeldetag : **16.04.81**

(54) **Kniegelenkbandage.**

(30) Priorität : 29.04.80 DE 3016426

(43) Veröffentlichungstag der Anmeldung :
04.11.81 Patentblatt 81/44

(45) Bekanntmachung des Hinweises auf die Patenterteilung : 26.10.83 Patentblatt 83/43

(84) Benannte Vertragsstaaten :
AT BE CH FR GB IT LI NL SE

(56) Entgegenhaltungen :
EP A 0 010 389
DE U 8 011 650
US A 2 858 540
US A 3 318 305
US A 3 786 804
US A 3 831 467
US A 3 926 186
US A 3 934 583
US A 4 084 584
US A 4 240 414

(73) Patentinhaber : **von Torklus, Detlef, Prof. Dr.**
**Jungfrauenthal 20**
**D-2000 Hamburg 13 (DE)**

**Thum, Oskar**
**Bebelallee 116**
**D-2000 Hamburg 60 (DE)**

**Wilharm, Friedrich**
**Martinistrasse 52**
**D-2000 Hamburg20 (DE)**

(72) Erfinder : **von Torklus, Detlef, Prof. Dr.**
**Jungfrauenthal 20**
**D-2000 Hamburg 13 (DE)**
Erfinder : **Thum, Oskar**
**Bebelallee 116**
**D-2000 Hamburg 60 (DE)**
Erfinder : **Wilharm, Friedrich**
**Martinistrasse 52**
**D-2000 Hamburg20 (DE)**

(74) Vertreter : **Minetti, Ralf, Dipl.-Ing.**
**Ballindamm 15**
**D-2000 Hamburg 1 (DE)**

Kniegelenkbandage

Die Erfindung betrifft eine Kniegelenkbandage mit einem konisch sich nach unten hin verjüngenden Kniestrumpf aus einem elastischen Material, der eine querverlaufende Halterung aufweist, die einen Stützriemen trägt. Mittels einer derartigen Bandage soll eine Entlastung der erkrankten Gleitfläche der Kniescheibe (patella) erreicht werden. Es kann sich dabei um knorpelige Mißbildungen handeln, wie auch allgemein um Abnutzungen oder Knorpelauflösung. Liegt infolge einer Deformierung nicht mehr eine glatte Gleitfläche vor, so werden bei der Bewegung des Knies und insbesondere der Kniescheibe, Schmerzen hervorgerufen.

Bei einer derartigen, aus der US-A-3 318 305 bekannten Kniegelenkbandage dient die als ein Riemen ausgebildete querverlaufende Halterung dem festen Sitz des Kniestrumpfes am Bein. Zu einer Stützung der Kniegelenkscheibe trägt sie nicht bei. Eine solche Stützung der Kniegelenkscheibe ist auch nicht vorgesehen. Der Kniestrumpf weist vielmehr eine Öffnung im Bereich der Kniescheibe auf, so daß diese in ihrer Bewegung unbehindert bleibt.

Die US-A-3 926 186 zeigt eine Muskelstützbandage, die aus einem breiten Stützriemen besteht. Für den Fall seiner Verwendung im Kniebereich ist der Stützriemen im oberen Randabschnitt mit einer Ausnehmung versehen, die der Kniescheibe in Größe und Form angepaßt ist, damit der Träger der Muskelbandage nicht im Gehen behindert ist.

Aufgabe der Erfindung ist es, eine Kniegelenkbandage zu schaffen, durch die beim Gehen und allgemein beim Bewegen des Knies die Kniescheibe durch eine Abstützung von unten her entlastet wird, um dadurch eine Linderung der Beschwerden bei bestimmten Knieerkrankungen und in Frühfällen eine Ausheilung zu erreichen. Gemäß der Erfindung ist dafür vorgesehen, daß sich die Halterung auf der Vorderseite des Kniestrumpfes etwa in mittlerer Höhe befindet und der Stützriemen die Kniescheibe von unten her abstützt. Dabei kann sich der Stützriemen in eine Grube bzw. in eine Einsenkung zwischen dem unteren Pol der Kniescheibe und dem Schienbeinknochen über dem Kniescheibenband einlegen und die Kniescheibe von unten her in einer angehobenen Stellung abstützen und bei einer Bewegung des Kniegelenkes in der angehobenen Stellung halten.

Dabei wird auf dem Kniescheibenband punktuell ein Druck ausgeübt. Durch ein derartiges Anheben der Kniescheibe wird diese in ihrem oberen Bereich vom Kniegelenk abgehoben und dadurch wird die Reibung im Bereich der Gleitfläche vermindert. Der Kniestrumpf sorgt dabei für die bleibende Lage des Stützriemens. Der Stützriemen kann dafür in dem Kniestrumpf eingearbeitet sein. Vorteilhafter ist es jedoch, wenn die Halterung aus einer rohrförmigen Führung besteht durch die sich der Stützriemen hindurch erstreckt, wobei die Halterung vorzugsweise aus dem gleichen Material wie der Kniestrumpf selber besteht, wobei für einen Waschvorgang des Knie-Strumpfes der Stützriemen aus der Führung herausgezogen werden kann.

Der Stützriemen ist vorzugsweise in seinem Stützabschnitt, das heißt in seinem Mittelabschnitt, wulstartig ausgebildet. Er kann dafür beispielsweise aus einer Umhüllung bestehen, die mit einer Füllung versehen ist, beispielsweise aus Filz oder einem anderen flexiblen Material.

Die Endabschnitte des Stützriemens werden mit Verschlußmitteln versehen, vorteilhafterweise mit einem Klettenbandverschluß, damit der Riemen in verschiedenen Längen angelegt werden kann, und geignet ist zum Tragen von Personen mit unterschiedlichen Wadendurchmessern. Um seine feste Lage zu gewährleisten, werden die Endabschnitte vorzugsweise aus einem dehnbaren Material hergestellt. Sie bestehen deshalb beispielsweise aus einem Gummiband.

Als nützlich hat es sich darüber hinaus gezeigt, wenn die Endabschnitte des Stützriemens bezogen auf den Stützabschnitt schräg nach unten ausgerichtet sind, damit der Stützriemen nicht zur Anlage in der Kniekehle kommt, sondern darunter und dadurch nicht die Gehbewegung behindert.

Der Stützabschnitt des Stützriemens selber kann nach unten durchgebogen ausgebildet sein, um eine größere Anpassung an die unten gewölbte Kniescheibe und damit eine breitere Anlage zu erhalten.

Ein Ausführungsbeispiel der Erfindung ist nachstehend unter Bezugnahme auf eine Zeichnung erläutert. In der Zeichnung zeigen:

Figur 1 Eine Kniegelenkbandage in der Draufsicht.

Figur 2 Den Stützriemen in der Draufsicht.

Figur 3 Den Stützabschnitt vom Stützriemen im Querschnitt.

Figur 4 Eine schematische Wiedergabe des Kniegelenkes mit angelegter Kniegelenkbandage.

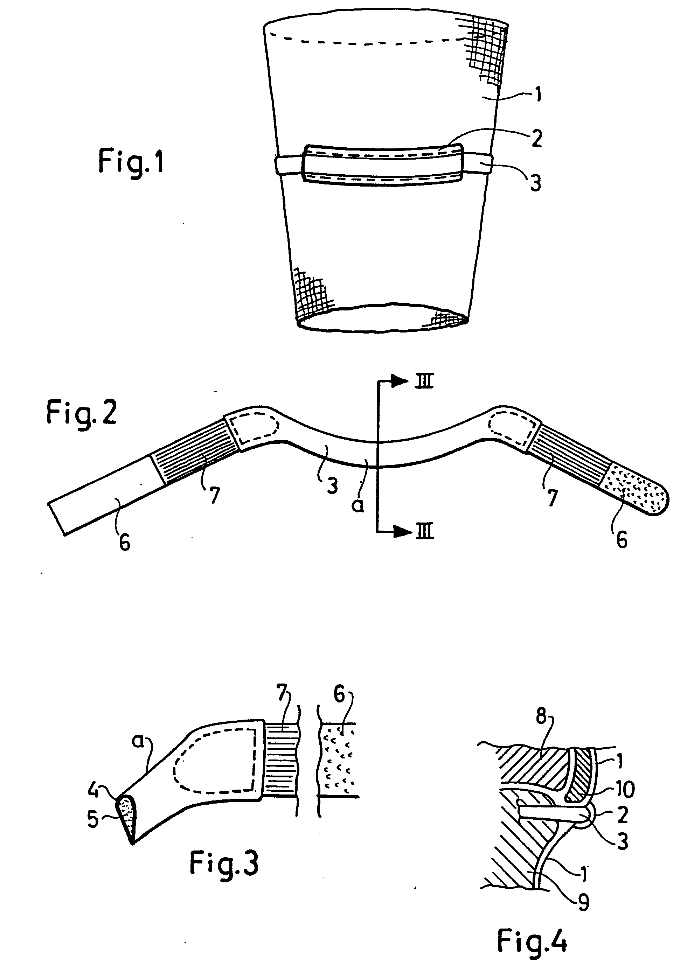

Die Zeichnung zeigt in Figur 1 eine Kniegelenkbandage mit einem bekannten Kniestrumpf 1, der aus einem elastischen Gewebe besteht, wie einem Gummigewebe, und demzufolge in allen Richtungen dehnbar ist. Der rohrförmige Kniestrumpf 1 ist nach unten hin sich konisch verjüngend ausgebildet, um dem Umstand Rechnung zu tragen, daß der Oberschenkel regelmäßig einen größeren Durchmesser hat als der Unterschenkel.

Auf den Kniestrumpf 1 ist eine als rohrförmige Führung ausgebildete Halterung 2 aufgenäht, die vorzugsweise aus dem gleichen Material besteht, wie der Kniestrumpf 1 selber. Sie kann jedoch beispielsweise auch aus Leder bestehen. Durch diese Halterung 2 hindurch erstreckt sich ein Stützriemen 3, der in seinem mittleren Stützabschnitt a nach unten gewölbt ausgebildet ist. Dieser Mittelabschnitt a wird überdeckt von der Halterung 2. Er besteht aus einer Lederumhüllung 4, die unterseitig zusammengenäht ist und eine Füllung 5 aus Filz oder einem anderen Material umschließt. Die Umhüllung 4 kann aber auch ebenfalls aus dem gleichen Material bestehen, wie der Kniestrumpf 1. Der mittlere Abschnitt a ist entsprechend der rohrförmigen Halterung 2 nach unten durchgekrümmt ausgebildet, um eine möglichst breite Stütze zu schaffen.

Die Endabschnitte des Stützriemens 3 sind als ein Klettenbandverschluß 6 in bekannter Art und Weise gestaltet. Zwischen diesen Endabschnitten und dem mittleren Stützabschnitt a ist der Stützriemen 3 mit dehnbaren Abschnitten 7 versehen, die in der Art eines Gummibandes ausgebildet sind, um den Stützriemen in seiner wirksamen Länge den jeweils vorliegenden Bedürfnissen anzupassen.

Bei Ingebrauchnahme der Kniegelenkbandage wird der Kniestrumpf 1 über den Fuß und den Unterschenkel soweit nach oben gezogen, daß sich der Stützriemen 3 in eine rinnenförmige Grube unterhalb der Kniescheibe 10 gemäß Figur 4 legt. Der Kniestrumpf 1 bedeckt dabei den Oberteil des Unterschenkelknochens 9 (Tibia) und den unteren Abschnitt des Oberschenkelknochens 8 (Femur). Bei einer Bewegung wird die Kniescheibe 10 durch den Stützriemen 3 in der angehobenen Stellung gehalten. Dadurch erfolgt fortlaufend eine Entlastung.

**Ansprüche**

1. Kniegelenkbandage mit einem konisch sich nach unten hin verjüngenden Kniestrumpf (1) aus einem elastischen Material, der eine querverlaufende Halterung (2) aufweist, die einen Stützriemen (3) trägt, dadurch gekennzeichnet, daß sich die Halterung (2) auf der Vorderseite des Kniestrumpfes (1) etwa in mittlerer Höhe befindet und der Stützriemen (3) die Kniescheibe (10) von unten her abstützt.

2. Kniegelenkbandage nach Anspruch 1, dadurch gekennzeichnet, daß die Halterung (2) als eine rohrförmige Führung ausgebildet ist, durch die sich der Stützriemen (3) erstreckt.

3. Kniegelenkbandage nach Anspruch 1, dadurch gekennzeichnet, daß die Halterung (2) aus dem gleichen Material wie der Kniestrumpf (1) besteht.

4. Kniegelenkbandage nach einem der vorhergehenden Ansprüche, dadurch gekennzeichnet, daß der Stützriemen (3) im Stützabschnitt (a) wulstartig ausgebildet ist.

5. Kniegelenkbandage nach einem der vorhergehenden Ansprüche, dadurch gekennzeichnet, daß der Stützriemen (3) aus einer Umhüllung (4) mit einer Füllung (5) besteht.

6. Kniegelenkbandage nach einem der vorhergehenden Ansprüche, dadurch gekennzeichnet, daß der Stützriemen (3) endseitig mit einem Klettenbandverschluß (6) versehen ist.

7. Kniegelenkbandage nach einem der vorhergehenden Ansprüche, dadurch gekennzeichnet, daß die Endabschnitte (6, 7) des Stützriemens (3) breiter sind als der Stützabschnitt (a).

8. Kniegelenkbandage nach einem der vorhergehenden Ansprüche, dadurch gekennzeichnet, daß der Stützabschnitt (a) des Stützriemens (3) nach unten durchgebogen ausgebildet ist.

9. Kniegelenkbandage nach einem der vorhergehenden Ansprüche, dadurch gekennzeichnet, daß die an den Stützabschnitt (a) anschließenden Riemenabschnitte (6, 7) im Winkel schräg nach unten ausgerichtet sind.

10. Kniegelenkbandage nach einem der vorhergehenden Ansprüche, dadurch gekennzeichnet, daß Abschnitte (7) des Stützriemens (3) aus einem dehnbaren Material bestehen.

**Claims**

1. Knee-joint bandage with a downwards tapered knee-sock (1) made of an elastic material, which shows a transverse holding device (2) which carries a supporting strap (3), characterised in that the holding device (2) is to be found on the face of the knee-sock (1) approximately in the middle of its height and that the supporting strap (3) supports the knee-cap (10) from below.

2. Knee-joint bandage according to claim 1, characterised in that the holding device (2) is formed as a tubular guide in which the supporting strap (3) extends.

3. Knee-joint bandage according to claim 1, characterised in that the holding device (2) consists of the same material as the knee-sock (1).

4. Knee-joint bandage according to one of the preceding claims, characterised in that the part of support (a) of the supporting strap (3) is formed into a pad.

5. Knee-joint bandage according to one of the preceding claims, characterised in that supporting strap (3) consists of a coating (4) with a filling (5) in it.

6. Knee-joint bandage according to one of the preceding claims, characterised in that the supporting strap (3) is provided with a Velcro strip fastener (6) on its extremity.

7. Knee-joint bandage according to one of the preceding claims, characterised in that the terminal parts (6, 7) of the supporting strap (3) are wider than the part of support (a).

8. Knee-joint bandage according to one of the preceding claims, characterised in that the part of support (a) of the supporting strap (3) is sagged.

9. Knee-joint bandage according to one of the preceding claims, characterised in that the parts of the strap (6, 7) which are adjacent to the supporting part (a) are aligned so as to be squarely inclined.

10. Knee-joint bandage according to one of the preceding claims, characterised in that some parts (7) of the supporting strap (3) are made of an extensible material.

**Revendications**

1. Bandage pour l'articulation du genou comportant une genouillère (1) se rétrécissant en cône vers le bas, en matière élastique, qui présente une attache transversale (2) qui porte une courroie de maintien (3), caractérisé en ce que l'attache (2) se trouve sur le devant de la genouillère (1) à peu près à mi-hauteur et que la courroie de maintien (3) soutient la rotule (10) par le bas.

2. Bandage pour l'articulation du genou selon la revendication 1, caractérisé en ce que l'attache (2) est formée comme un guide tubulaire dans lequel s'étend la courroie de maintien (3).

3. Bandage pour l'articulation du genou selon la revendication 1, caractérisé en ce que l'attache (2) est composée de la même matière que la genouillère (1).

4. Bandage pour l'articulation du genou selon l'une quelconque des revendications précédentes, caractérisé en ce que la courroie de maintien (3) est matelassée à la façon d'un bourrelet dans la section de maintien (a).

5. Bandage pour l'articulation du genou selon l'une quelconque des revendications précédentes, caractérisé en ce que la courroie de maintien (3) se compose d'une gaine (4) et d'un rembourrage (5).

6. Bandage pour l'articulation du genou selon l'une quelconque des revendications précédentes, caractérisé en ce que la courroie de maintien (3) est pourvue à son extrémité d'une fermeture en bande Velcro (6).

7. Bandage pour l'articulation du genou selon l'une quelconque des revendications précédentes, caractérisé en ce que les sections finales (6, 7) de la courroie de maintien (3) sont plus larges que la section de maintien (a).

8. Bandage pour l'articulation du genou selon l'une quelconque des revendications précédentes, caractérisé en ce que la section de maintien (a) de la courroie de maintien (3) est fléchie vers le bas.

9. Bandage pour l'articulation du genou selon l'une quelconque des revendications précédentes, caractérisé en ce que les sections de courroie (6, 7) faisant suite à la section de maintien (a) sont orientées en équerre de façon oblique vers le bas.

10. Bandage pour l'articulation du genou selon l'une quelconque des revendications précédentes, caractérisé en ce que les sections (7) de la courroie de maintien (3) sont en matière extensible.

0 039 010

Fig.1

Fig.2

Fig.3

Fig.4